# EUROPEAN PATENT APPLICATION

(11) **EP 4 684 728 A1**
(43) Date of publication of application: **28.01.2026**
(21) Application number: 25190855.4
(22) Date of filing: 22.07.2025
(51) Int. Cl.: A61B 5/107, A61B 5/287, A61B 5/367, A61B 5/00

(54) **SYSTEM AND METHOD FOR FAR-FIELD VOLTAGE MAPPING FOR SCAR SEVERITY ESTIMATION**

(30) Priority: 23.07.2024 US 202463674324 P; 16.06.2025 US 202519238791
(71) Applicant: Biosense Webster (Israel) Ltd., 2066717 Yokneam (IL)
(72) Inventor: RODRIGUEZ, Haim, 2066717 Yokneam (IL); PFEFFER, Shmuel Yitzhak, 2066717 Yokneam (IL); TSOREF, Liat, 2066717 Yokneam (IL)
(74) Representative: Carpmaels & Ransford LLP

(57) **Abstract**

The present disclosure provides a method and system for analyzing unipolar electrophysiological (EP) signals acquired by a multi-electrode catheter placed in a ventricle of a patient's heart. The method includes receiving unipolar EP signals, extracting unipolar far-field signals from the unipolar EP signals, and analyzing the extracted unipolar far-field signals to estimate the distribution of scar regions across a thickness of wall tissue of the ventricle. Using the estimated distribution, a unipolar far-field EP map showing the scar regions is generated. The method further includes displaying the unipolar far-field EP map including the scar regions to a user. The system comprises a display device and a processor configured to perform the steps of the method.

## Description

### CROSS-REFERENCE TO RELATED APPLICATION

This application claims the benefit of U.S. Provisional Patent Application 63/674,324, filed July 23, 2024, which is incorporated herein by reference.

### FIELD OF THE DISCLOSURE

This disclosure relates generally to electrophysiological (EP) signals, and specifically to the evaluation of electrical propagation in the heart.

### BACKGROUND OF THE DISCLOSURE

Estimation of non-local cardiac electrophysiological signals was previously proposed in the patent literature. For example, U.S. Patent 11,464,437 describes a medical analysis system that includes at least one catheter to be inserted into a body-part having a tissue surface, and comprising sensing electrodes to contact and receive electrical signals from the tissue surface, and processing circuitry to receive unipolar signals from individual ones of the plurality of the sensing electrodes, compute a combined far-field and mid-field signal based on summing and filtering ones of the received unipolar signals received from at least a pair of sensing electrodes disposed around a point of interest, compute a far-field signal as a weighted average of the received unipolar signals, weighted according to respective distances of the sensing electrodes from the point of interest, and compute and output a mid-field signal, representative of electrical activity below the tissue surface at the point of interest, based on subtracting the computed far-field signal from the computed combined far-field and mid-field signal.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electrophysiology (EP) pacing, mapping, and ablation system, according to an example of the present disclosure;
Fig. 2 is a diagram that schematically illustrates a unipolar far-field EP map of cardiac scar severity in a wall tissue of left ventricle tissue, according to an example of the present disclosure;
Fig. 3 is a flow chart schematically describing a method to generate a far-field unipolar EP map of cardiac scar severity, according to an example of the present disclosure;
Fig. 4 is a diagram that schematically describes the method to extract far-field from unipolar signals acquired using a multi-electrode catheter, according to an example of the present disclosure; and
Fig. 5 is a set of graphs of unipolar signals acquired using the multi-electrode catheter of Fig. 4, and the respectively extracted far-field signals, according to an example of the present disclosure.

### DETAILED DESCRIPTION OF EXAMPLES

### OVERVIEW

Cardiac electrophysiological (EP) catheter mapping is a technique that produces a three-dimensional EP map of the inner surface of cardiac chamber wall tissue, such as a unipolar activation voltage map of a left ventricle (LV). Medical professionals can use the EP map to determine the precise source location of an arrhythmia or, when performing a medical procedure such as cardiac ablation, determine where to apply treatment (e.g., isolation).

In EP mapping of the inner surface of cardiac wall tissue, far-field cardiac EP signals are considered interference that degrades the EP map quality. Far-field signals are presumed to occur due to electrical activity originating further away from the catheter electrodes. Far-field signals can distort or obscure local electrical activity, i.e., distort signals originating in the immediate proximity of the electrodes.

However, in thick cardiac wall tissue, such as the LV wall, electricity is conducted throughout the different layers of the endocardium and myocardium. Some medical conditions of a ventricle require estimating the electrical activity beneath the tissue surface. For example, after performing an ablation (from the endocardial or the epicardial side), residual EP conduction in an ablated ventricle tissue region may indicate that ventricular tachycardia (VT) persists due to insufficient ablation.

While the above-described localized cardiac EP mapping method may identify electrically inactive (e.g., scar) tissue mostly located on the inner tissue surface (i.e., an endocardial scar), it cannot easily assess the depth of the detected scar. Moreover, in some cases, the inner surface may be electrically active with scarred tissue located only underneath, e.g.**,** deep in a myocardium wall tissue or on the epicardial surface, which localized mapping will have difficulty assessing.

The above-described insufficient EP diagnostic capability may sometimes be mitigated using cardiac MRI, which can provide a tomographic assessment of thick ventricle wall tissue (e.g., identifying healthy or scarred tissue over wall thickness). However, an MRI scan is costly, has limited availability, and cannot be promptly followed up with catheter treatment if such is required.

Examples of the present disclosure described herein provide a catheter EP mapping technique using far-field signals to estimate the distribution of scar regions in thick cardiac walls, such as the LV wall. The disclosed method uses the fact that the EP far-field signals drop slightly differently for different scar types when the catheter electrode moves from healthy tissue toward a significant scar boundary or border line.

The disclosed technique estimates whether a scar is present in endocardial tissue or affects intracardial and/or epicardial tissue. The technique provides a measure to estimate scar severity by comparing the scar thickness to the entire thickness of the cardiac wall tissue. Finally, the technique provides a unipolar far-field EP map that describes the EP properties of wall tissue both laterally and over its thickness.

In one example, the disclosed method analyzes unipolar far-field signals extracted using the method described in U.S. Patent Application Publication 2023/0181087, which is assigned to the assignee of the present disclosure. The analysis of the extracted unipolar far-field signals aims to estimate the distribution of scar regions across a thickness (e.g., over an entire thickness) of ventricle wall tissue.

In cases where an endocardial scar does not manifest any significant far-field signal drop toward the scar border line, the disclosed method estimates that most of the tissue under the scar is healthy. In cases where no endocardial scar appears but a significant far-field signal drop occurs, the disclosed method estimates a significant intracardial or epicardial scar is present.

The method defines scar severity as the normalized ratio of scar width to total tissue width. It estimates the ratio based on (i) a mathematical model that analyzes the far-field signal and/or spatial derivatives and (ii) correspondences obtained empirically between the unipolar far-field EP signals and anatomical dissections or MRI imaging of actual scars.

Using the above-described analysis of far-field signals, a processor generates a unipolar far-field EP map that visualizes the distribution of scar regions. In one example, the unipolar far-field EP map visually represents the different behaviors of scar-related peak-to-peak far-field potentials. In another example, the unipolar far-field EP map visually represents the different behaviors of scar-related voltage gradients, such as a distribution of maximal values of negative derivatives of the far-field signal, which change differently for different scar depths. During technique development, the above-mentioned empirical correspondence is used to validate the diagnostic power of the disclosed far-field-signal-based EP maps.

Finally, the disclosed method can have the processor delineate the borders of the endocardial scar regions and overlay the border lines on the disclosed unipolar far-field EP map. Displaying such borders can ease the visualization of scar regions along different depths of the cardiac wall tissue.

A clinician using the disclosed unipolar far-field EP map of scar severity can promptly be guided (e.g., without depending on cardiac MRI diagnostics) to a better clinical strategy for treating EP aberrant thick myocardium tissue, such as that of the LV.

### SYSTEM DESCRIPTION

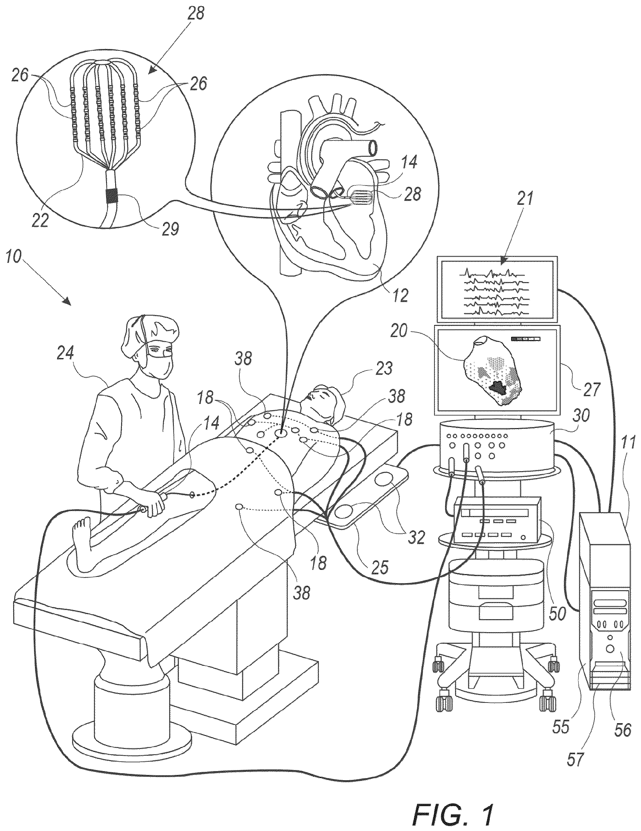

Fig. 1 is a schematic, pictorial illustration of a catheter-based electrophysiology (EP) pacing, mapping, and ablation system 10, according to an example of the present disclosure.

System 10 includes a flat multi-electrode EP mapping catheter 14 (e.g., an OPTRELL^{™} catheter made by Biosense-Webster), which physician 24 percutaneously inserts via a sheath through the patient's vascular system into the left ventricle of heart 12. Physician 24 brings a distal end assembly 28 of catheter 14 into contact with the heart wall to sense EP signals (e.g., unipolar signals) over a given area of heart 12 of patient 23.

Catheter 14 includes a large-area flat distal end assembly 28 that carries multiple electrodes 26 over a plurality of splines 22**.** Catheter 14 may include a position sensor 29, embedded in or near distal end assembly 28 on a shaft 46 of catheter 14, for tracking its position and orientation of distal end assembly 28. Optionally, and preferably, position sensor 29 is a magnetic-based position sensor including three magnetic coils for sensing three-dimensional (3D) position and orientation.

Magnetic-based position sensor 29 may be operated with a location pad 25 that includes a plurality of magnetic coils 32 configured to generate magnetic fields in a predefined working volume. The real-time position of distal end assembly 28 of catheter 14 may be tracked based on magnetic fields generated with location pad 25 and sensed by magnetic-based position sensor 29. Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 and impedance-based tracking of electrodes 26 and for sensing unipolar signals. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays cardiac signals 21 (e.g., electrograms from a 12-lead ECG device acquired with body surface ECG electrodes 18). Recorder 11 may include pacing capability to pace the heart rhythm and/or may be electrically connected to a standalone pacer.

The patient interface unit (PIU) 30 is an interface configured to establish electrical communication between catheters, electrophysiological equipment, power supply, and a workstation 55 to control system 10 operation and receive EP signals from the catheter or apply pacing signals. Electrophysiological equipment of system 10 may include, for example, multiple catheters, location pad 25, body surface ECG electrodes 18, electrode patches 38, an ablation energy generator 50, and a recorder 11. Optionally, and preferably, PIU 30 additionally includes processing capability for implementing real-time computations of catheter locations and for performing ECG calculations.

Workstation 55 includes memory 57, a processor 56 unit with memory or appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27, such as places where ablation energy has been applied. One commercial product embodying elements of system 10 is available as the CARTO^{™} 3 System, available from Biosense Webster, Inc., 31A Technology Drive, Irvine, CA 92618.

In some examples, processor 56 typically comprises a general-purpose computer programmed in software to carry out the functions described herein. The software may be downloaded to the computer in electronic form over a network, for example, or it may, alternatively or additionally, be provided and/or stored on non-transitory tangible media, such as magnetic, optical, or electronic memory.

System 10 may include an ablation energy generator 50 adapted to conduct ablative energy to one or more electrodes at a distal end tip of a catheter configured for ablation. The energy produced by ablation energy generator 50 may include, but is not limited to, radiofrequency (RF) energy or pulsed-field ablation (PFA) energy, including monopolar or bipolar high-voltage DC pulses, to be used to effect irreversible electroporation (IRE), or combinations thereof.

For ablation, physician 24 similarly brings a distal end of an ablation catheter to a target site. One or more additional catheters can be inserted via the sheath. They may include a catheter for sensing intracardiac electrogram signals, a catheter dedicated to ablating, and/or a catheter dedicated to both EP mapping and ablating.

This configuration of system 10 is shown by way of example to illustrate certain problems addressed by examples of the present disclosure and to demonstrate the application of these examples in enhancing the performance of such a system. However, examples of the present disclosure are not limited to this specific example system, and the principles described herein may similarly be applied to other medical systems. For example, multi-electrode catheter types, such as the multi-arm OCTARAY^{™} catheter or a basket catheter, may be used.

### FAR-FIELD EP SCAR SEVERITY MAP

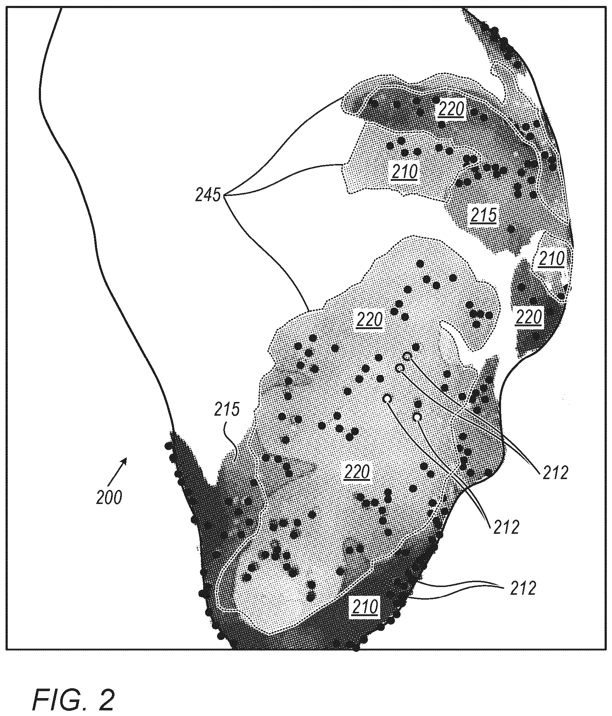

Fig. 2 is a schematic, pictorial rendering of a far-field unipolar EP map 200 of scar severity in a wall tissue of a left ventricle, according to an example of the present disclosure. Map 200 may be produced and displayed, for example, by processor 56 of system 10.

In the present example, scar regions 210 include endocardial tissue, scar regions 220 include intracardial tissue, and scar regions 215 include epicardial tissue. In some cases, scar regions 210, 215, and 220 partially overlap.

To differentiate scar regions 210, 215, and 220, processor 56 may display them with distinct graphical indications, such as distinct colors or grey levels (as shown) or patterns (not shown).

Fig. 2 further displays endocardial scar region 210 borders 245 as obtained from a localized unipolar EP map using method steps 310 and 324 described below.

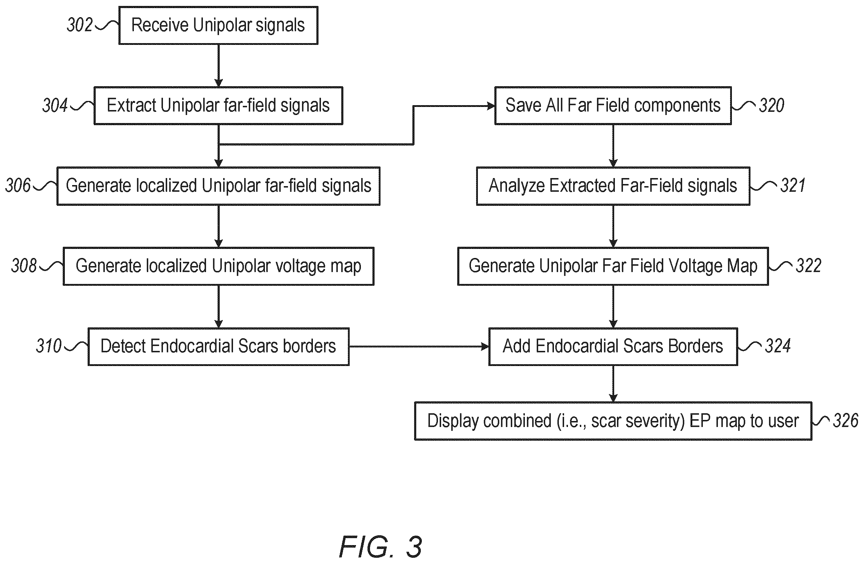

Fig. 2 may also show some of the locations, denoted 212, where the catheter electrode acquired the unipolar signals themselves. Processor 56 generated EP map 200 from data points acquired at such locations using the method described in Fig. 3. Note that the graphical presentation of data points 212 on the map is not part of the disclosed algorithm. Therefore, showing data-points 212 is left to the user's discretion, meant to provide the user with an additional, rawer representation of the acquired data.

Fig. 2 describes the disclosed concept purely by example. Other suitable graphical representations, such as a graphical encoding according to the region involved (e.g., if only a single scar region 210, 215, or 220, or a pair of scar regions (e.g., 210, 215) or (215, 220), or all three scar regions (210, 215, 220) are present about a given LV location), may be constructed in alternative examples.

### METHOD OF FAR-FIELD EP MAPPING FOR SCAR SEVERITY ESTIMATION

Fig. 3 is a flow chart schematically describing a method to generate a far-field unipolar EP map of cardiac scar severity, according to an example of the present disclosure. The algorithm, according to the presented example, carries out a process that begins with a processor receiving unipolar signals acquired by a multi-electrode catheter, such as catheter 14 of Fig. 1, at a unipolar signal receiving step 302. The processor may be processor 56 or any processor of an offline computer. The signals may have been acquired some time ago and are uploaded from a memory, such as memory 57 or an offline memory.

At unipolar far-field signals extraction step 304, processor 56 extracts from the unipolar signals received at step 302 the unipolar far-field signals using the method described in U.S. Patent Application Publication 2023/0181087.

In generating localized unipolar signals step 306, the processor uses the unipolar far-field signals to generate clean localized unipolar signals.

In the disclosed technique, the processor also saves the far-field signals for use with the disclosed algorithm at a far-field signal-saving step 320.

Using the localized unipolar signals cleaned in step 306, the processor generates a localized unipolar voltage map in a localized EP map-generating step 308.

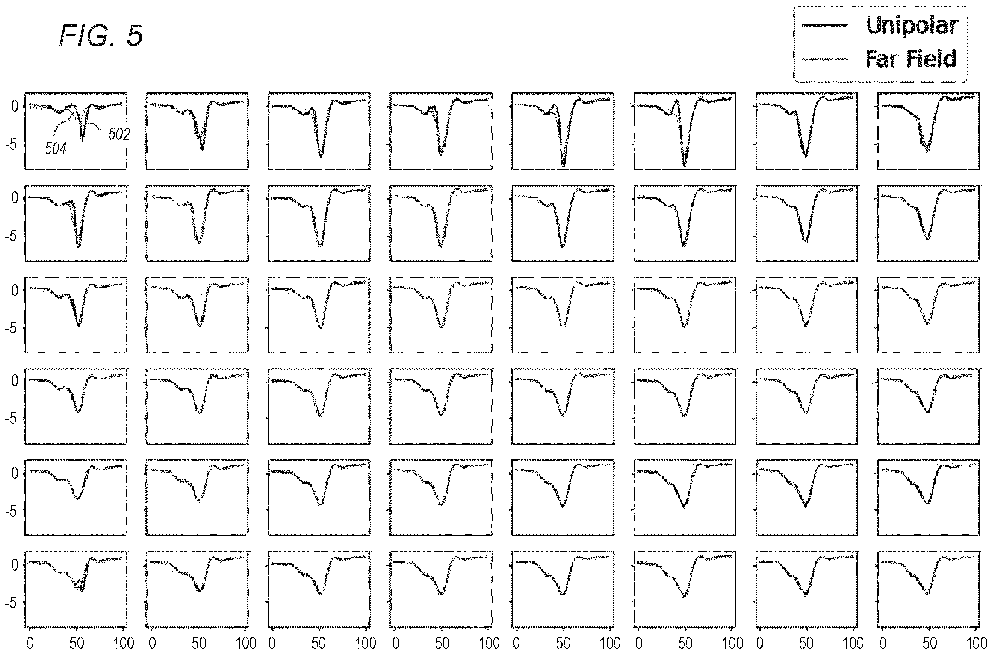

In parallel, at an analysis step 321, the processor analyzes the unipolar far field signals saved in step 320 to estimate scar locations and depths. The processor relies on the far-field signals being at certain wall tissue locations slightly different (e.g., smaller) than the total signal as a function of location and depth, as seen in Fig. 5 below. The method defines scar severity as the normalized ratio of scar width to total tissue width. It estimates the ratio based on (i) a mathematical model that analyzes the far-field signal and/or spatial derivatives and (ii) correspondences obtained empirically between the unipolar far-field EP signals and anatomical dissections or MRI imaging of actual scars.

Using the analysis done in step 321, the processor generates a far-field unipolar EP map 200, in a far-field EP map generating step 322. The EP map may be a voltage map or a voltage gradient map, to name two examples.

In border detection step 310, the processor detects the endocardial scar regions borders 345 on the localized map.

The processor adds (e.g., overlays) borders 345 on far-field unipolar EP map 200, at adding scar borders step 324. The resulting map is named in this disclosure "scar severity map."

Finally, the processor displays the combined map (i.e., the scar severity map) to a user, e.g., on display device 27.

The flowchart of Fig. 3 is provided as an example. Additional steps may be included, such as displaying the localized EP map of step 308 to the user.

### UNIPOLAR FAR-FIELD SIGNALS EXTRACTION

One method to estimate and remove unipolar far-field signals is described in U.S. Patent Application Publication 2023/0181087, which is assigned to the assignee of the present disclosure. The algorithm disclosed in U.S. Patent Application Publication 2023/0181087 is inspired by an assumption that intracardiac unipolar far-field cardiac EP signals are similar but not the same on all multi-electrode EP mapping catheter electrodes. In the technique described therein, a mapping engine (e.g., a processor running the algorithm) weights catheter electrodes in opposite proportion to distances to identify a common signal (e.g., a far field signal) component across the electrodes. The mapping engine uses This common signal component to reduce or cancel far-field interference precisely. Reducing or canceling the far-field interference by the mapping engine produces cleaner near-field signals for improved localized (e.g., near-field) cardiac mapping, as described in the above-mentioned patent applications which are assigned to the assignee of the present disclosure.

The presently disclosed technique uses the far-field signals alone to electro-physiologically estimate scar distribution in thick cardiac walls (e.g., including intracardial and epicardial portions), such as the LV. The technique may utilize the localized EP map to overlay endocardial scar region borders on the disclosed unipolar far-field EP map.

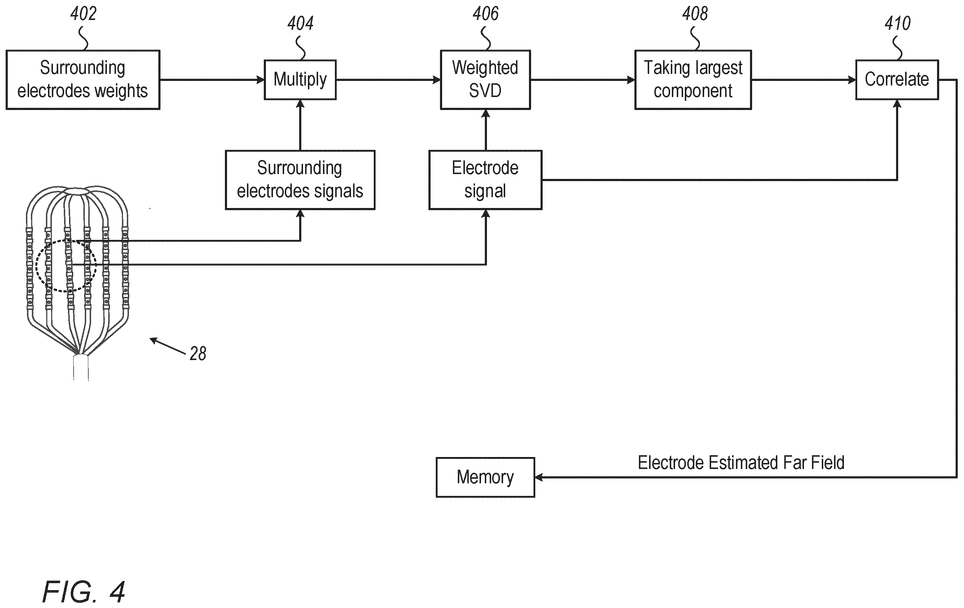

Fig. 4 is a diagram that schematically describes the method to extract far-field from unipolar signals acquired using a multi-electrode catheter, such as the OPTRELL^{™} catheter, according to an example of the present disclosure. In step 402, a processor weights the electrodes surrounding a specific electrode in opposite proportion to a distance from that specific electrode. In step 404, the processor multiplies the electrodes far-field signals by the respective weights. A matrix of the weighted far-field signals contribution for each electrode is generated (step 406) and the largest signal is extracted (step 408). The processor correlates (step 410) the largest signal with the electrode signal to provide the far field estimation per the electrode in question.

Fig. 5 is a set of graphs of unipolar signals 502 acquired using the multi-electrode of Fig. 4 (such as the OPTRELL^{™} catheter), and the respectively extracted far-field signals 504, according to an example of the present disclosure. As seen, in some tissue locations, the extracted far-field amplitudes are smaller than the overall unipolar signal amplitude, due to the extracted signals 504 being scar sensitive.

### CLINICAL VALIDATION OF FAR-FIELD EP MAP OF SCAR SEVERITY

One way to validate the EP map is to establish correspondence between scarred animal hearts maintained alive in a lab and measuring and analyzing the far field EP signals in the hearts. A cohort of the living animal hearts includes, for example, three hearts A, B and C, each prepared with a different scar type: Endocardium, Epicardium and Myocardium. For each heart, the same three wall tissue regions are chosen, having large, medium and small relative tissue thickness. A mapping catheter such as the above mentioned OPTRELL^{™} catheter acquires a reference signal of the healthy animal hearts.

Subsequently, for each of hearts A, B, an C location, an approximately 2 cm diameter round endocardium or epicardium or myocardium scar is ablated with transmurality ratio varied using multiple ablations (or any other technique). A 10 sec signal recording is taken after a 0.25 transmurality ratio is reached while placing the catheter symmetrically above the scar. Ablation is applied until a 0.5 transmurality ratio is reached followed by a 10 sec signal recording. Ablation is applied until a 0.75 transmurality ratio is reached followed by a 10 sec signal recording. All unipolar acquired signals would be processed for Far-field signal extraction and analysis.

### EXAMPLES

### Example 1

A method involves receiving unipolar EP signals acquired by a multi-electrode catheter (14) placed in a ventricle (12) of a heart of a patient (23). From these unipolar EP signals, unipolar far-field signals are extracted. The extracted unipolar far-field signals are analyzed to estimate the distribution of scar regions (210, 215, 220) across a thickness of wall tissue of the ventricle. Using this estimated distribution, a unipolar far-field EP map (200) showing the scar regions is generated. The unipolar far-field EP map including the scar regions is then displayed to a user on a display device (27).

### Example 2

The method according to Example 1, wherein analyzing the extracted unipolar far-field signals comprises analyzing one or more spatial derivatives of the far-field signals.

### Example 3

The method according to Example 1 or 2, wherein generating the unipolar far-field EP map comprises showing epicardial scars (215) on the unipolar far-field EP map.

### Example 4

The method according to any of Examples 1-3, wherein generating the unipolar far-field EP map comprises showing intracardial scars (220) on the unipolar far-field EP map.

### Example 5

The method according to any of Examples 1-4, wherein generating the unipolar far-field EP map comprises calculating, over the scar regions, a ratio of scar thickness to an entire thickness of the wall tissue, and wherein displaying the unipolar far-field EP map comprises displaying the ratio on the EP map.

### Example 6

The method according to any of Examples 1-5, and comprising generating from the unipolar EP signals a localized unipolar voltage map.

### Example 7

The method according to Example 6, and comprising detecting endocardial scar borders (245) in the localized unipolar voltage map and superimposing the detected borders on the unipolar far-field EP map.

### Example 8

The method according to any of Examples 1-7, and comprising displaying the localized unipolar voltage map to the user.

### Example 9

The method according to any of Examples 1-8, wherein the multi-electrode catheter is one of a flat catheter and a multi-arm catheter.

### Example 10

A system includes a display device (27) and a processor (56). The processor is configured to receive unipolar EP signals acquired by a multi-electrode catheter (14) placed in a ventricle (12) of a heart of a patient (23). The processor extracts from the unipolar EP signals unipolar far-field signals and analyzes the extracted unipolar far-field signals to estimate the distribution of scar regions (210, 215, 220) across a thickness of wall tissue of the ventricle. Using the estimated distribution, the processor generates a unipolar far-field EP map (200) showing the scar regions and displays the unipolar far-field EP map including the scar regions to a user on the display device.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A method, comprising:
receiving unipolar EP signals acquired by a multi-electrode catheter (14) placed in a ventricle (12) of a heart of a patient (23);
extracting from the unipolar EP signals unipolar far-field signals;
analyzing the extracted unipolar far-field signals to estimate a distribution of scar regions (210, 215, 220) across a thickness of wall tissue of the ventricle;
using the estimated distribution, generating a unipolar far-field EP map (200 showing the scar regions; and
displaying the unipolar far-field EP map including the scar regions to a user.

2. The method according to claim 1, wherein analyzing the extracted unipolar far-field signals comprises analyzing one or more spatial derivatives of the far-field signals.

3. The method according to claim 1 or claim 2, wherein generating the unipolar far-field EP map comprises showing epicardial scars (215) on the unipolar far-field EP map.

4. The method according to any of claims 1 to 3, wherein generating the unipolar far-field EP map comprises showing intracardial scars (220) on the unipolar far-field EP map.

5. The method according to any of claims 1 to 4, wherein generating the unipolar far-field EP map comprises calculating, over the scar regions, a ratio of scar thickness to an entire thickness of the wall tissue, and wherein displaying the unipolar far-field EP map comprises displaying the ratio on the EP map.

6. The method according to any of claims 1 to 5, and comprising generating from the unipolar EP signals a localized unipolar voltage map, and optionally comprising detecting endocardial scar borders (245) in the localized unipolar voltage map and superimposing the detected borders on the unipolar far-field EP map.

7. The method according to any of claims 1 to 6, and comprising displaying the localized unipolar voltage map to the user.

8. A system, comprising:
a display device (27); and
a processor (56), which is configured to:
receive unipolar EP signals acquired by a multi-electrode catheter (14) placed in a ventricle (12) of a heart of a patient (23);
extract from the unipolar EP signals unipolar far-field signals;
analyze the extracted unipolar far-field signals to estimate a distribution of scar regions (210, 215, 220) across a thickness of wall tissue of the ventricle;
using the estimated distribution, generate a unipolar far-field EP map (200) showing the scar regions; and
display the unipolar far-field EP map including the scar regions to a user on the display device.

9. The system according to claim 8, wherein analyzing the extracted unipolar far-field signals comprises analyzing one or more spatial derivatives of the far-field signals.

10. The system according to claim 8 or claim 9, wherein generating the unipolar far-field EP map comprises showing epicardial (215) scars on the unipolar far-field EP map.

11. The system according to any of claims 8 to 10, wherein generating the unipolar far-field EP map comprises showing intracardial scars (220) on the unipolar far-field EP map.

12. The system according to any of claims 8 to 11, wherein generating the unipolar far-field EP map comprises calculating, over the scar regions, a ratio of scar thickness to an entire thickness of the wall tissue, and wherein displaying the unipolar far-field EP map comprises displaying the ratio on the EP map.

13. The system according to any of claims 8 to 12, wherein the processor is further configured to generate from the unipolar EP signals a localized unipolar voltage map, optionally wherein the processor is further configured to detect endocardial scar borders (245) in the localized unipolar voltage map and superimpose the detected borders on the unipolar far-field EP map.

14. The system according to any of claims 8 to 13, wherein the processor is further configured to display the localized unipolar voltage map to the user.

15. The method according to any of claims 1 to 7 or the system according to any of claims 8 to 14, wherein the multi-electrode catheter is one of a flat catheter and a multi-arm catheter.
